# EUROPEAN PATENT APPLICATION

(11) **EP 4 431 032 A1**
(43) Date of publication of application: **18.09.2024**
(21) Application number: 22891879.3
(22) Date of filing: 03.11.2022
(51) Int. Cl.: A61B 17/22

(54) **SHOCK WAVE ELECTRODE ASSEMBLY, BALLOON CATHETER APPARATUS, AND MEDICAL DEVICE**

(30) Priority: 09.11.2021 CN 202111323375
(71) Applicant: Shanghai Bluesail Boyuan Medical Technology Co., Ltd., Shanghai 200131 (CN)
(72) Inventor: XU, Pengfei, Shanghai 200131 (CN); CUI, Yuhu, Shanghai 200131 (CN); QUE, Zhiwen, Shanghai 200131 (CN); WANG, Cheng, Shanghai 200131 (CN)
(74) Representative: Viering, Jentschura & Partner mbB Patent- und Rechtsanwälte
(86) International application number: PCT/CN2022/129425
(87) International publication number: WO 2023/083084

(57) **Abstract**

The present disclosure provides a shock wave electrode assembly, comprising an inner electrode, an outer electrode, and an insulating layer located between the inner electrode and the outer electrode. A surface of the inner electrode is provided with a raised pin. The insulating layer is provided with a first hole, and the outer electrode is provided with a second hole. A diameter of the second hole is greater than that of the first hole. The raised pin, the first hole, and the second hole are arranged coaxially. The raised pin extends through the first hole and into the second hole. The present disclosure also provides a balloon catheter apparatus and a medical device. The present disclosure improves a threshold pulse voltage of an electrode, so that high-intensity shock waves can be efficiently generated, thereby fracturing a calcified lesion in a blood vessel and effectively improving treatment efficacy for an occlusive lesion.

## Description

For all purposes, the present application claims priority of China Patent application No. 202111323375.8, filed on November 9, 2021, the entire disclosure of which is incorporated herein by reference as part of the present application.

### TECHNICAL FIELD

The present disclosure relates to an electrode assembly, in particular to a shock wave electrode assembly, a balloon catheter device, and a medical apparatus.

### BACKGROUND

Atherosclerosis is a disease with arterial stenosis and hardening caused by plaque accumulation. The plaque is composed of fibrous tissue, fat, and calcium. The accumulated calcified plaque hinders the normal flow of blood and reduces the supply of oxygen and nutrients to the body, thereby causing diseases related to the arteries supplying blood to key parts of the body (including the brain, heart and limbs).

Traditional angioplasty often uses balloon catheter to physically expand lesions (such as calcified lesions) and make blood vessels unblocked again. However, when the balloon expands, it is easy to cause tearing and damage to the adventitia of the blood vessel. The method of electrohydraulic effect is used to destroy the structure of the calcified lesion attached to the diseased blood vessel wall. That is, electrodes are arranged in an angioplasty-type balloon, and can be used for the operations on calcified lesions in the arterial wall by working with the high-voltage generator. In this kind of operations, the balloon catheter is pushed into the blocked area along a guide wire, and then the balloon is pressurized with the conductive fluid to make the balloon pressed against the blood vessel. A series of high-voltage pulses are applied to the electrodes in the balloon by a high-voltage generator. Each pulse passes through the electrodes to generate micro bubbles in the conductive fluid. These bubbles grow and burst instantly to generate shock waves. The shock waves travel through the balloon wall and reach the blocked area, which ruptures the calcified plaque. Once the calcified plaque ruptures, the balloon can be further expanded to unblock the blood vessels. Repeated pulses can destroy the structure of calcified lesions and expand narrowed blood vessels without damaging the surrounding soft tissues, which can avoid the problem of blood vessel wall damage caused by balloon expansion during traditional angioplasty. However, the electrode usually needs a relatively high pulse voltage to reach the breakdown voltage of the conductive fluid in the circuit and generate shock waves. This relatively high pulse voltage also raises the requirements for the internal circuit design of the high-voltage generator.

### SUMMARY

The present disclosure provides a shock wave electrode assembly, a balloon catheter device, and a medical apparatus, which, for example, lower the requirements of the shock wave electrode assembly for pulse voltage, can efficiently generate high-intensity shock waves, rupture the calcified lesion in the blood vessel, and improve the therapeutic effect on blocked lesions.

The present disclosure provides a shock wave electrode assembly, which includes an inner electrode, an outer electrode, and an insulating layer between the inner electrode and the outer electrode, a surface of the inner electrode is provided with a protruding pin, the insulating layer is provided with a first hole, the outer electrode is provided with a second hole, a diameter of the second hole is larger than that of the first hole, and the protruding pin, the first hole, and the second hole are configured such that the protruding pin extends through the first hole and into the second hole.

According to the embodiment of the present disclosure, the protruding pin arranged on the surface of the inner electrode extends through the first hole and into the second hole, so that an annular gap is formed between the outer electrode and the protruding pin, and the said gap allows the formation of a discharge circuit between the outer electrode and the inner electrode. When the shock wave electrode assembly is placed into a liquid and given an appropriate pulse voltage, the breakdown voltage of the filled liquid can be reached, an electric spark can be emitted, and shock waves can be generated. Common shock wave electrode assemblies usually need relatively high pulse voltages to cause breakdown of the conductive fluid in the circuit, so as to generate shock waves. This relatively high pulse voltage also raises the requirements for the internal circuit design of the high-voltage generator. According to the embodiment of the present disclosure, the design of the shock wave electrode assembly with the protruding pin structure can lower the requirements of the shock wave electrode assembly for pulse voltage, that is, the shock wave electrode assembly can generate shock waves at a lower pulse voltage. The safety level is improved by using the said shock wave electrode assembly. In addition, when the same pulse voltage is given, compared with a common shock wave electrode assembly, the shock wave electrode assembly with the protruding pin structure shows higher electric energy density, thus being able to generate shock waves with higher energy. This will be beneficial to destroying the structure of the calcified lesion attached to the diseased blood vessel wall and improving the therapeutic effect on blocked lesions.

In one embodiment, the inner electrode is in a sheet configuration, the insulating layer and the outer electrode are in annular configurations, the outer electrode and the insulating layer are coaxially arranged, and the outer electrode is sleeved on the insulating layer, and the protruding pin, the first hole and the second hole are coaxially arranged.

In one embodiment, a radial dimension of the first hole is equal to the maximum radial dimension of the protruding pin, so that the protruding pin is fixed in the first hole.

In one embodiment, a height of the protruding pin extending in the second hole is 1/3 to 2/3 of a thickness of the outer electrode. The shock wave electrode assembly with the said height can generate the largest electric energy density, so that the effectiveness of the shock wave electrode assembly can be maximized.

In one embodiment, the protruding pin is cylindrical, frustum-shaped or conical. According to the actual use needs, the corresponding shape of the protruding pin is adopted to have the discharge mode and energy of the electrodes varied.

In one embodiment, a ratio of a thickness of the inner electrode to a thickness of the outer electrode is in a range from 1: 1 to 5: 1. In one embodiment, the ratio of the thickness of the insulating layer to the thickness of the inner electrode is in a range from 1: 1 to 1: 2. If the insulating layer is thinner, the distance between the inner electrode and the outer electrode will be too close, and electrical conduction will be formed as the breakdown voltage is easily reached. If the insulating layer is thicker, the distance between the inner electrode and the outer electrode will be too far to achieve effective breakdown. The thickness of the insulating layer is related to the dielectric constant of the insulating material. The insulating layer having high dielectric constant has good insulating performance, and under the same circumstances, the use of such relatively thin insulating layer can meet the requirements.

In one embodiment, a ratio of a diameter of the first hole to a diameter of the second hole is in a range from 1: 2 to 1: 5. By adjusting the ratio of the diameter of the first hole to the diameter of the second hole, the direct contact between the two electrodes due to the circumferential rotation of the outer electrode relative to the insulating layer can be avoided to a certain extent.

The present disclosure further provides a balloon catheter device including a balloon, an inner tube, an outer tube, and any one of the shock wave electrode assemblies according to the embodiments of the present disclosure, a distal end of the inner tube extends through the balloon and is connected with a distal end of the balloon, and the shock wave electrode assembly is arranged on an exterior of the inner tube, and the outer tube is sleeved on the inner tube and connected with a proximal end of the balloon.

According to the embodiment of the present disclosure, the above shock wave electrode assembly is arranged inside the balloon catheter device used for angioplasty, so that shock waves can be efficiently generated, the calcified lesions in the blood vessel can be ruptured, and the therapeutic effect on blocked lesions can be improved.

In one embodiment, the balloon catheter device further includes a lead wire extending along an axial direction of the balloon catheter device, the lead wire includes a first lead wire and a second lead wire, the first lead wire is connected with the inner electrode, and the second lead wire is connected with the outer electrode. In one embodiment, the balloon catheter device includes a plurality of shock wave electrode assemblies arranged at intervals along the axial direction of the inner tube. By using a plurality of the shock wave electrode assemblies, the balloon catheter device is capable of treating multiple calcified lesions simultaneously, thus saving the operation time and improving efficiency.

In one embodiment, a plurality of shock wave electrode assemblies are arranged in series. In one embodiment, the plurality of the shock wave electrode assemblies are arranged in the same circumferential direction of the inner tube, or are arranged at an angle in the circumferential direction. By adjusting the relative arrangement direction between shock wave electrode assemblies, the intensity and distribution of shock waves generated by the entire balloon are improved.

The present disclosure further provides a medical apparatus, which includes a high-voltage generator and any one of the balloon catheter devices according to the embodiments of the present disclosure, and a proximal end of the lead wire of the balloon catheter device is connected with the high-voltage generator, a pulse voltage of the high-voltage generator is in a range from 500 V to10 kV, a pulse voltage width is in a range from 200 ns to 20 µs, a pulse current of the high-voltage generator is in a range from 50 A to 400 A, and the pulse current width is in a range from 10 ns to 2 µs.

In one embodiment, an intensity of the sound pressure of the shock wave generated by each of the shock wave electrode assemblies is in a range from 2 MPa to 20 MPa, and the discharge frequency is in a range from 0.1 Hz to 10 Hz.

### BRIEF DESCRIPTION OF DRAWINGS

In order to more clearly illustrate the technical solutions of the embodiments of the present disclosure, the drawings of the embodiments will be briefly introduced below. Obviously, the drawings in the following description only relate to some embodiments of the present disclosure and do not limit the present disclosure.
Figs. 1A to 1C are schematic cross-sectional views of shock wave electrode assemblies according to some embodiments of the present disclosure.
Fig. 2 is a schematic perspective view of a shock wave electrode assembly according to one embodiment of the present disclosure.
Fig. 3 is an exploded view of the shock wave electrode assembly of Fig. 2.
Fig. 4 is a comparison diagram of the threshold voltage between a shock wave electrode assembly according to an embodiment of the present disclosure and a common shock wave electrode assembly.
Fig. 5 is a curve graph showing the electric energy density of a shock wave electrode assembly according to an embodiment of the present disclosure and the electric energy density of a common shock wave electrode assembly under different pulse voltages, respectively.
Fig. 6 is a diagram showing the relationship between the height position of the protruding pin and the electric energy density of the electrodes.
Fig. 7 is a schematic diagram of a balloon catheter device according to one embodiment of the present disclosure.
Figs. 8A to 8C are schematic cross-sectional views of balloon catheter devices according to some embodiments of the present disclosure.
Figs. 9A to 9C are schematic diagrams of a balloon catheter device having a plurality of shock wave electrode assemblies according to some embodiments of the present disclosure.

### DETAILED DESCRIPTION

The technical solutions in the embodiments of the present disclosure will be clearly and completely described below with reference to the accompanying drawings in the embodiments of the present disclosure. Obviously, the described embodiments are only some of the embodiments of the present disclosure, rather than all of the embodiments. Based on the embodiments in present disclosure, all other embodiments obtained by those skilled in the art without making creative efforts fall within the scope of protection of present disclosure.

Unless otherwise defined, all terms (including technical and scientific terms) used in the embodiments of present disclosure have the same meaning as commonly understood by those skilled in the art to which present disclosure belongs. It should also be understood that terms such as those defined in general dictionaries should be interpreted as having meanings consistent with their meanings in the context of the related art, and should not be interpreted in an idealized or extremely formal sense, unless explicitly defined by the embodiment of the present disclosure.

The words "first", "second" and similar words used in the embodiments of present disclosure do not indicate any order, quantity or importance, but are only used to distinguish different components. Similar words such as "a", "an" or "the" do not mean a quantity limit, but mean that there is at least one. Similarly, similar words such as "include", "including", "comprise", or "comprising" mean that the elements or objects appearing before the word cover the elements or objects listed after the word and their equivalents, without excluding other elements or objects. Similar words such as "connected" or "connecting" are not limited to physical or mechanical connection, but may include electrical connection or communication connection, whether direct or indirect. The "radial dimension" in the present invention refers to the distance between the farthest two points on the cross section of an element or an object. Taking the case where the cross section is a circle as an example, the radial dimension is the diameter. The "thickness" in the present invention refers to the distance between the upper and lower opposite surfaces of an object, and it should be noted that "the thickness of the inner electrode" in the present invention refers to the distance between the upper and lower opposite surfaces of the inner electrode, and this distance does not include the height of the protruding pin.

Traditional angioplasty often uses balloon catheter to physically expand lesions (such as calcified lesions) and make blood vessels unblocked again. However, when the balloon expands, it is easy to cause tearing and damage to the adventitia of the blood vessel.

The inventors of the present disclosure have found that in some existing solutions, the electrodes are placed in an angioplasty balloon, and the electrodes work together with a high-voltage generator, which can be used for calcified lesions treatment in the arterial wall. In this type of operation, the balloon catheter is pushed into the blocked area of the blood vessel along the guide wire, and then the balloon is pressurized with conductive fluid to make the balloon pressed against the blood vessel. A series of high-voltage pulses are applied to the electrodes in the balloon by a high-voltage generator. Each pulse passes through the electrodes to generate micro bubbles in the conductive fluid. These bubbles grow and burst instantly to generate shock waves. The shock waves pass through the balloon wall, reach the blocked area of the blood vessel, and rupture the calcified plaque. Once the calcified plaque ruptures, the balloon can be further expanded to unblock the blood vessels.

The design of electrode configuration is very important for the generation of the shock waves, which not only enables the formation of bubbles, but also not hinders the movement of bubbles, thereby facilitating the generation of shock waves. The inventors of the present disclosure have also found that in other existing solutions, different electrode structures are disclosed. Ring-plate structures are widely used, which usually include inner electrodes, insulating layers and outer electrodes. The inner and outer electrodes have a ring structure and are arranged coaxially, and the insulating layer is arranged between the inner electrode and the outer electrode. When the high voltage pulse passes through the electrodes, the inner electrode, the outer electrode and the conductive fluid form a circuit for generating the shock waves. However, this electrode configuration usually requires a relatively high pulse voltage to cause the breakdown of the conductive fluid in the circuit and generate shock waves. This relatively high pulse voltage also raises the requirements for the internal circuit design of the high-voltage generator.

Therefore, the shock wave electrode assembly and balloon catheter device provided by the embodiments of the present disclosure solve some of the above technical problems, which can be specifically described below.

The invention provides a shock wave electrode assembly, which includes an inner electrode, an outer electrode, and an insulating layer located between the inner electrode and the outer electrode. A surface of the inner electrode is provided with a protruding pin, the insulating layer is provided with a first hole, the outer electrode is provided with a second hole, the diameter of the second hole is larger than that of the first hole, the protruding pin, the first hole and the second hole are configured such that the protruding pin extends through the first hole and into the second hole.

According to the embodiment of the present disclosure, the protruding pin arranged on the surface of the inner electrode extends through the first hole and into the second hole, so that an annular gap is formed between the outer electrode and the protruding pin, and the gap allows the formation of a discharge circuit between the outer electrode and the inner electrode. When the shock wave electrode assembly is placed into the liquid and given an appropriate pulse voltage, the breakdown voltage of the filled liquid can be reached, an electric spark can be emitted, and shock waves can be generated. Common shock wave electrode assemblies usually require relatively high pulse voltages to obtain a shock wave. According to the embodiment of the present disclosure, the design of the shock wave electrode assembly with the protruding pin structure can lower the requirements of the shock wave electrode assembly on pulse voltage, that is, the shock wave electrode assembly can generate shock waves at a lower pulse voltage, thereby improving the safety of using the shock wave electrode assembly. In addition, when the same pulse voltage is given, compared with a common shock wave electrode assembly, the shock wave electrode assembly with the protruding pin structure shows higher electric energy density, thus being able to generate shock waves with higher energy, which will be beneficial to destroying the structure of the calcified lesion attached to the diseased blood vessel wall and improving the therapeutic effect on blocked lesions.

Embodiments of the present disclosure and examples thereof will be described in detail with reference to the accompanying drawings.

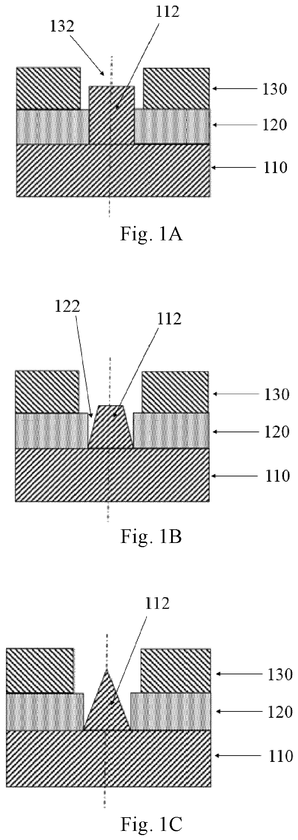

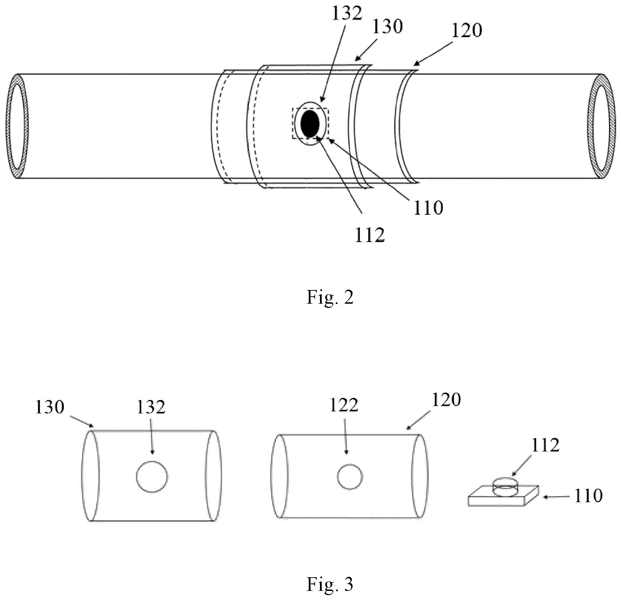

Figs. 1A to 1C are schematic cross-sectional views of shock wave electrode assembly according to some embodiments of the present disclosure. Fig. 2 is a schematic perspective view of a shock wave electrode assembly according to one embodiment of the present disclosure. Fig. 3 is an exploded view of the shock wave electrode assembly of Fig. 2.

As shown in Figs. 1A to 1C, the shock wave electrode assembly 100 includes an inner electrode 110, an outer electrode 130, and an insulating layer 120 located between the inner electrode 110 and the outer electrode 130. The surface of the inner electrode 110 is provided with a protruding pin 112, and the insulating layer 120 is provided with a first hole 122. The outer electrode 130 is provided with a second hole 132, and a diameter of the second hole 132 is larger than that of the first hole 122. The protruding pin 112 extends through the first hole 122 and into the second hole 132, so that a discharge circuit is formed between the inner electrode 110 and the outer electrode 130.

In one embodiment, the inner electrode 110 has a sheet configuration, and the outer electrode 130 and the insulating layer 120 are in annular configurations, as shown in Figs. 2 and 3. The outer electrode 130 and the insulating layer 120 are coaxially arranged, and the outer electrode 130 is sleeved on the insulating layer 120. The inner diameter of the outer electrode 130 matches with the outer diameter of the insulating layer 120, thereby reducing the possibility of relative movement.

In one embodiment, the ratio of the thickness of the inner electrode to the thickness of the outer electrode is in a range from 1: 1 to 5: 1. In one embodiment, the ratio of the thickness of the insulating layer to the thickness of the inner electrode is in a range from 1: 1 to 1: 2. According to the embodiment of the present disclosure, the insulating layer mainly functions as a barrier between the inner electrode and the outer electrode. If the insulating layer is thinner, the distance between the inner electrode and the outer electrode will be too close, and electrical conduction will be formed as the breakdown voltage is easily reached. If the insulating layer is relatively thicker, the distance between the inner electrode and the outer electrode will be too far to achieve effective breakdown state. Therefore, for the embodiment of the present disclosure, for example, the thickness ratio of inner electrode: insulating layer: outer electrode may be 1:1:1. In addition, the thickness of the insulating layer is related to the dielectric constant of the insulating material. The insulating layer having a high dielectric constant has a good insulating performance, and under the same circumstances, a thinner insulating layer can meet the requirements. For example, for polyamide material with high dielectric constant, the thickness ratio of inner electrode: insulating layer: outer electrode can be 2:1:2. Therefore, according to the actual situation, insulating materials with different dielectric constants can be used to make insulating layers, and the thickness ratio of inner electrode: insulating layer: outer electrode will change accordingly. The materials of the outer electrode and the inner electrode can be conductive metals, such as stainless steel, copper, silver and the like. The material of the insulating layer is non-conductive insulating substance, such as polyimide, polytetrafluoroethylene, polyethylene, polypropylene and other polymers.

In the embodiment of the present disclosure, once the length of the outer electrode 130 is too long, the passage convenience of the balloon catheter will be affected. Therefore, the length of the outer electrode 130 can be adjusted accordingly as required, which is not limited by the present disclosure. According to the embodiment of the present disclosure, the length of the outer electrode 130 is shorter than that of the insulating layer 120, so that the outer electrode can be better fixed on the insulating layer 120.

In the embodiment of the present disclosure, the outline of structure of the outer electrode 130 may be, for example, elliptical, oval, rectangular, triangular, irregular and other geometric structures. During the actual use of the shock wave electrode assembly 100, the outer electrode 130 needs to be sleeved on the insulating layer 120 and form a discharge circuit with the inner electrode 110.

In one embodiment, the protruding pin 112, the first hole 122, and the second hole 132 can also be arranged coaxially, so that a uniform annular gap is formed between the outer electrode and the protruding pin, which is beneficial to uniform discharge. In one embodiment, the ratio of the diameter of the first hole 122 to the diameter of the second hole 132 is in a range from 1: 2 to 1: 5. On the one hand, the direct contact between the two electrodes due to the axial rotation of the outer electrode relative to the insulating layer can be avoided, that is, to ensure that a discharge circuit is formed between the inner electrode and the outer electrode; on the other hand, sufficient discharge effect can be ensured, that is, on the premise that the applied voltage between the inner and outer electrodes exceeds the breakdown voltage of the medium, as the distance between the inner and outer electrodes increases, the energy accumulated between the electrodes becomes higher, the shock wave intensity is greater. Therefore, the appropriate diameter ratio can be adopted according to the shock wave intensity needed for the actual design.

According to an embodiment of the present disclosure, the radial dimension of the first hole 122 is equal to the maximum radial dimension of the protruding pin 112, so that the protruding pin 112 is fixed in the first hole 122, and thus the inner electrode 110 is connected with the insulating layer 120. The first hole 122 plays a role in fixing the position of the protruding pin 112 of the inner electrode 110. If the outer surface of the protruding pin 112 of the inner electrode 110 is separated from the wall of the first hole 122, the protruding pin 112 of the inner electrode 110 is easily not coaxially arranged with the second hole 132 of the outer electrode 130, which affects the actual discharge effect. In addition, the protruding pin 112 will slide relative to the second hole 132 of the outer electrode 130, which will affect the reliability of the shock wave electrode assembly 100.

In one embodiment, the structure of the protruding pin 112 of the inner electrode 110 may be cylindrical (as shown in Fig. 1A), frustum-shaped (as shown in Fig. 1B) or conical (as shown in Fig. 1C), or may be other structures, preferably cylindrical. The shape of the protruding pin 112 can affect the discharge mode and energy of the electrodes. Using cylindrical protrusion can not only reduce the threshold breakdown voltage, but also obtain a high electrical energy density. The present disclosure does not limit this, and may depend on the actual situation. According to the actual use, the distance of the gap between the inner electrode 110 and the outer electrode 130 can be adjusted as needed. Given the size of the first hole and the second hole, the above-mentioned distance of the gap can be adjusted by changing the shape of the protruding pin 112 of the inner electrode 110, thereby changing the discharge mode and energy of the electrodes. In another embodiment, the wall of the first hole of the insulating layer may have an inclined surface with a certain angle to ensure matching with the outer surface of the protruding pin with a frustum-shaped or conical structure. Therefore, the protruding pin can be better fixed on the insulating layer 120. It is ensured that the protruding pin 112 will not move relative to the first hole 122 of the insulating layer 120 during the actual operation of the shock wave electrode assembly 100.

According to the embodiment of the present disclosure, the protruding pin 112 is arranged on the surface of the inner electrode 110, and the protruding pin 112 extends through the first hole 122 and into the second hole 132, so that an annular gap is formed between the outer electrode 130 and the protruding pin 112, so as to form a discharge circuit between the outer electrode 130 and the inner electrode 110. When the shock wave electrode assembly 100 is placed into a liquid and given an appropriate pulse voltage, the breakdown voltage of the filled liquid can be reached, an electric spark can be emitted, and shock waves can be generated. The shock waves propagate through the liquid inside the balloon and impacts the balloon wall and calcified area. Repeated pulses can destroy the structure of calcified lesions and expand narrowed blood vessels without damaging the surrounding soft tissues, which can avoid the problem of blood vessel wall damage caused by balloon expansion in traditional angioplasty. A common shock wave electrode assembly usually needs relatively high voltages to reach the breakdown voltage of the conductive fluid in the circuit, so as to obtain a shock wave. Higher pulse voltages also lead to demanding internal circuit design of high-voltage generator.

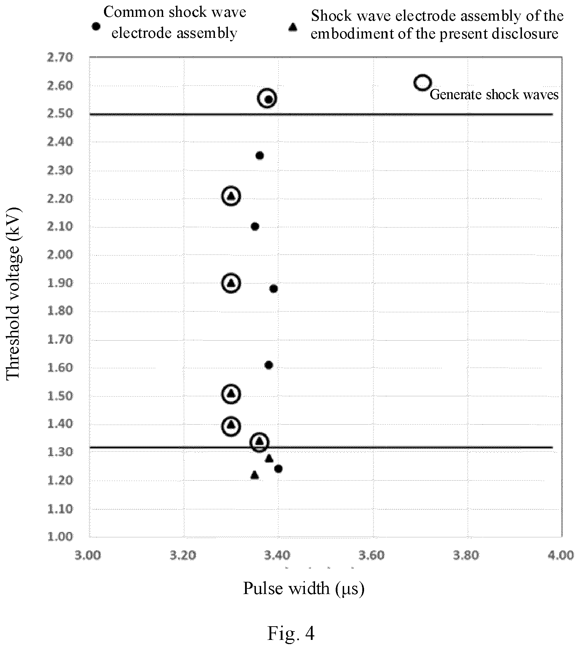

Fig. 4 is a comparison diagram of the threshold voltage of a shock wave electrode assembly according to an embodiment of the present disclosure and a common shock wave electrode assembly. As can be seen from the figure, when the voltage of the common shock wave electrode assembly exceeds 2.5KV, the electrode assembly begins to generate shock waves, that is, the threshold voltage of the common shock wave electrode assembly is determined to be 2.5kV. However, it can be found that the shock wave electrode assembly of the embodiment of the present disclosure begins to generate shock waves when its voltage exceeds 1.33KV. Compared with the common shock wave electrode assembly, the threshold voltage of the shock wave electrode assembly in the embodiment of the present disclosure is lower, being only 1.33 KV Therefore, it can be concluded that the protruding pin 112 provided on the surface of the inner electrode 110 of the embodiment of the present disclosure extends through the first hole 122 and into the second hole 132, an annular gap is formed between the outer electrode 130 and the protruding pin 112, and a discharge circuit is formed between the outer electrode 130 and the inner electrode 110. The design of the shock wave electrode assembly with the protruding pin structure lowers the requirement of the shock wave electrode assembly for pulse voltage, in other words, the shock wave electrode assembly of the embodiment of the present disclosure can generate shock waves at a lower pulse voltage, and the safety of using the shock wave electrode assembly is improved.

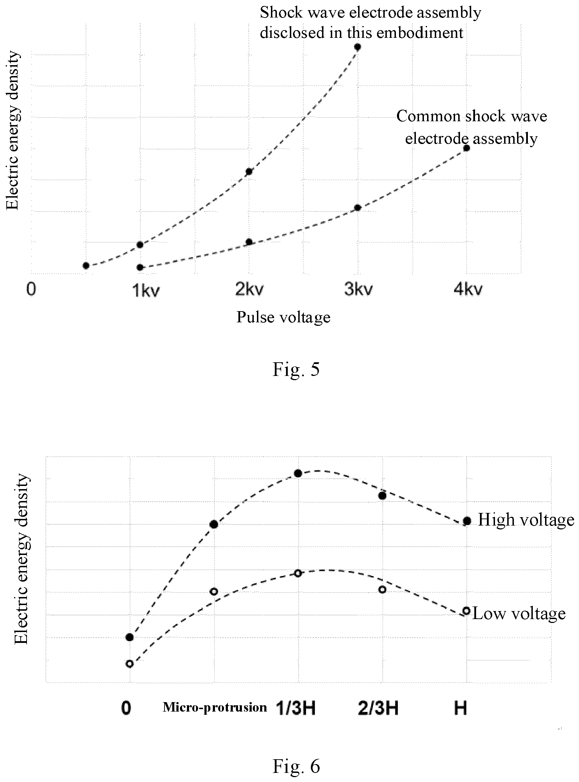

Fig. 5 is a curve graph showing the electric energy density of a shock wave electrode assembly according to an embodiment of the present disclosure and the electric energy density of a common shock wave electrode assembly under different pulse voltages, respectively. As can be seen from the figure, when the pulse voltage is in a range from 1 KV to 3 KV, if the same pulse voltage is applied to the two shock wave electrode assemblies, the electric energy density of the shock wave electrode assembly in the embodiment of the present disclosure is higher than that of the common shock wave electrode assembly, and the difference between them increases with the increase of pulse voltage. In other words, given the same pulse voltage, compared with the common shock wave electrode assembly, the shock wave electrode assembly with the protruding pin structure shows higher electric energy density, thus being able to generate shock waves with higher energy. This will be beneficial to destroying the structure of the calcified lesion attached to the diseased blood vessel wall and improving the therapeutic effect on blocked lesion.

In one embodiment, the height of the protruding pin 112 extending in the second hole 132 is 1/3 to 2/3 of the thickness of the outer electrode 130. According to the shock wave electrode assembly of the present disclosure, because the discharge position is the gap between the protruding pin 112 of the inner electrode 110 and the second hole 132, the height of the protruding pin 112 will affect the discharge mode and energy, and an appropriate height can exert the maximum utility of the electrode. Fig. 6 is a diagram showing the relationship between the height position of the protruding pin and the electric energy density of the electrode. As shown in Fig. 6, on the abscissa, "0" means that the height position of the protruding pin 112 is on the surface of the inner electrode 110, and the height of the protruding pin 112 is 0. "Micro-protrusion" means that the height of the protruding pin 112 starts with the surface of the insulating layer 120, and the height of the protruding pin 112 is equal to the thickness of the insulating layer. In other words, the distance from "0" to the "micro-protrusion" refers to the thickness of the annular insulating layer 120 between the inner electrode 110 and the outer electrode 130. While "1/3H", "2/3H" and "H" respectively indicate that the height position of the protruding pin 112 is 1/3 thickness position, 2/3 thickness position, and outer surface position of the outer electrode 130. As can be seen from the figure, with the increase of the height of the protruding pin 112, the change of the protruding pin in height position makes the electric energy density of the electrode show a trend of increasing first and then decreasing. And the trend is basically the same under the two test conditions of high voltage and low voltage. In addition, the data points are fitted, and the obtained fitting curve shows that the highest point of the electric energy density falls within the range of 1/3-2/3 of the abscissa, that is, the height of the protruding pin 112 extending in the second hole 132 is 1/3-2/3 of the thickness of the outer electrode 130. Under this condition, the electric energy density of the shock wave electrode assembly 100 is the highest, so that the shock wave electrode assembly 100 can exert its best effect.

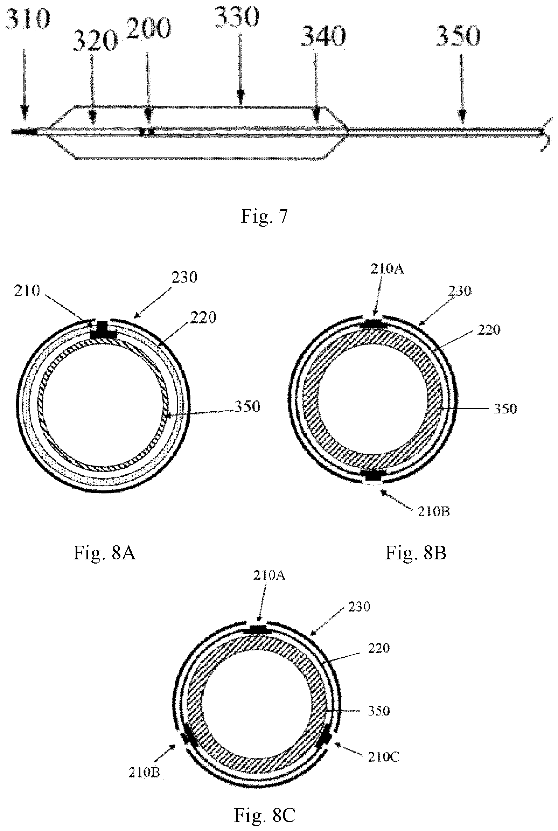

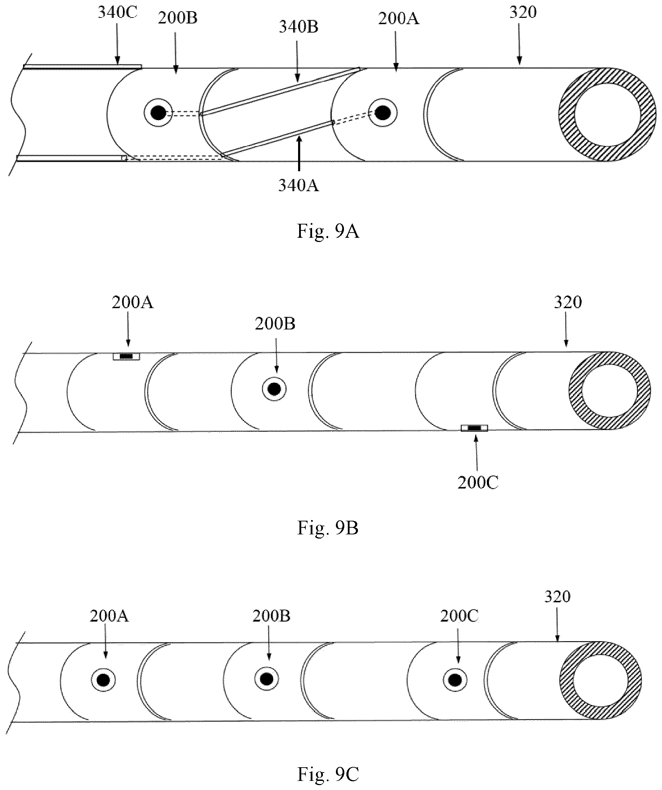

Fig. 7 is a schematic diagram of a balloon catheter device according to one embodiment of the present disclosure. As shown in Fig. 7, the balloon catheter device 310 includes an inner tube 320, a balloon 330, an outer tube 350, and a shock wave electrode assembly 200. The shock wave electrode assembly 200 includes an inner electrode 210, an outer electrode 230, and an insulating layer 220 located between the inner electrode 210 and the outer electrode 230. The surface of the inner electrode 210 is provided with a protruding pin 212, the insulating layer 220 is provided with a first hole 222, and the outer electrode 230 is provided with a second hole 232. The details of the shock wave electrode assembly have been described above and will not be described here. The distal end of the inner tube 320 extends through the balloon 330 and is connected with the distal end of the balloon 330, and the shock wave electrode assembly 200 is arranged on the outer surface of the inner tube 320. The outer tube 350 is sleeved on the inner tube 320 and connected with the proximal end of the balloon 330. In one embodiment, the balloon catheter device 310 further includes a lead wire 340 extending along the axial direction of the balloon catheter device 310, and the lead wire 340 includes a first lead wire 340A and a second lead wire 340B (see Fig. 9A), the first lead wire 340A is connected with the inner electrode 210, and the second lead wire 340B is connected with the outer electrode 230. The two lead wires extend along the axial direction of the balloon catheter device 310, and are connected with a high-voltage generator (not shown) through connecting lead wires M (not shown), respectively. The current is transmitted to the inner electrode 210 through the first lead wire 340A, and the inner electrode 210 forms a circuit with the outer electrode 230 through the filled conductive fluid. The high-voltage pulse causes the breakdown of the conductive fluid and generates shock waves in the axial direction of the first hole 222. Then, the current is transmitted to the outer electrode 230 and returned to the high-voltage generator along the second lead wire 340B.

According to the embodiment of the present disclosure, the surface of the inner electrode 210 is provided with the protruding pin 212, and a discharge circuit is formed between the outer electrode 230 and the protruding pin 212 of the inner electrode 210. When the shock wave electrode assembly 200 is placed into a liquid and given an appropriate pulse voltage, the breakdown voltage of the filled liquid can be reached, an electric spark can be emitted, and shock waves can be generated. The shock wave propagates through the liquid inside the balloon and impacts the balloon wall and calcified area. Repeated pulses can destroy the structure of calcified lesions and expand narrowed blood vessels without damaging the surrounding soft tissues, which can avoid the problem of blood vessel wall damage caused by balloon expansion in traditional angioplasty. The shock wave electrode assembly of the balloon catheter device in the embodiment of the present disclosure can generate shock waves at a lower pulse voltage, thereby improving the safety of using the balloon catheter device. In addition, under the condition of giving the same pulse voltage, compared with the common shock wave electrode assembly, the shock wave electrode assembly with the protruding pin structure shows higher electric energy density, so the balloon catheter device in the embodiment of the present disclosure can more effectively destroy the structure of the calcified lesion attached to the diseased blood vessel wall and improve the therapeutic effect on the blocked lesion through the generated shock waves with higher energy.

Fig. 8A is a schematic cross-sectional view of a balloon catheter device according to an embodiment of the present disclosure. The positional relationship of the components of the shock wave electrode assembly in the balloon catheter device is further given in combination with the assembling process of the inner electrode 210, the insulating layer 220, and the outer electrode 230. With reference to Fig. 3, Fig. 7 and Fig. 8A, in order to install the shock wave electrode assembly in the balloon catheter device, firstly, the inner electrode 210 is placed on the surface of the inner tube 320, then the insulating layer 220 with the first hole 222 is sleeved on the inner tube 320, and the insulating layer 220 is moved to align the first hole 222 with the protruding pin 212, and the protruding pin 212 is fixed in the first hole 222 by pressing, so that the inner electrode 210 can be fixed. The inner tube 320 and the insulating layer 220 can be further fixed with glue. Then, the outer electrode 230 is sleeved on the insulating layer 220, and the second hole 232 is aligned with the first hole 222 and the protruding pin 212, so that the protruding pin 212, the first hole 222, and the second hole 232 are coaxial, and the outer electrode 230 can be fixed on the insulating layer 220 with glue. In one embodiment, the outer electrode 230 includes a plurality of second holes 232, the insulating layer 220 includes a plurality of first holes 222, and the balloon catheter device includes a plurality of inner electrodes 210. The number of the inner electrodes 210, the number of the first holes 222, and the number of the second holes 232 are equal, so that the balloon catheter device can have a plurality of shock wave electrode assemblies. During the actual operation, there may be many calcified lesions to be treated. Therefore, a structure with multiple shock wave electrode assemblies can be used to construct multiple discharge regions by the balloon catheter device. On the one hand, the ability of the balloon catheter device to treat multiple calcified lesions at the same time can be improved, on the other hand, the uniformity of shock wave distribution in the circumferential space of the inner tube can be improved, which is beneficial to the treatment of calcified lesions. Figs. 8A to 8C show schematic cross-sectional views of balloon catheter devices provided with one, two or three shock wave electrode assemblies. In addition, the distribution of the shock wave electrode assembly in the circumferential direction of the inner tube 320 can be adjusted according to the actual position distribution of the calcified lesions to be treated, which is not limited by the present disclosure.

In one embodiment, the balloon catheter device 310 includes a plurality of shock wave electrode assemblies 200 arranged at intervals along the axial direction of the inner tube 320. If there are multiple calcified lesions to be treated, and they are separated by a certain distance, it is inconvenient to transport the balloon catheter device to different calcified lesions for treatment one by one in the case where the balloon catheter device adopts a single shock wave electrode assembly 200. Therefore, in view of the above situation, a balloon catheter device with a plurality of shock wave electrode assemblies 200 arranged at intervals along the axial direction of the inner tube can be adopted. Fig. 9A shows a schematic diagram of a balloon catheter device with two shock wave electrode assemblies 200. Meanwhile, the distribution of different shock wave electrode assemblies 200 in the axial direction of the inner tube 320 is adjusted according to the actual position distribution of the calcified lesions to be treated, which is not limited by the present disclosure.

In one embodiment, a plurality of shock wave electrode assemblies are arranged in series. A plurality of shock wave electrode assemblies 200 can be arranged in the balloon catheter device, and the shock wave electrode assemblies 200 are connected in series to generate shock waves in the axial direction of the first hole of each shock wave electrode assembly 200. The inside of the balloon can be filled with conductive fluid through the liquid passage cavity. The shock wave electrode assemblies 200 are arranged on the outer surface of the inner tube 320 at a certain distance, and transmits current by connecting a plurality of lead wires. The arrangement in series of the two shock wave electrode assemblies is shown in Fig. 9A. The first lead wire 340A is connected to the protruding pin of the inner electrode of the shock wave electrode assembly 200A which forms a circuit with the outer electrode of the shock wave electrode assembly 200A through the conductive fluid, and a first shock wave is generated in the axial direction of the first hole of the shock wave electrode assembly 200A. The second lead wire 340B is arranged to connect the outer electrode of the shock wave electrode assembly 200A with the inner electrode of the shock wave electrode assembly 200B, and the inner electrode of the shock wave electrode assembly 200B forms a circuit with the outer electrode of the shock wave electrode assembly 200B through the conductive fluid. A second shock wave is generated in the axial direction of the first hole of the shock wave electrode assembly 200B. The third lead wire 340C connected with the outer electrode of the shock wave electrode assembly 200B extends along the axial direction of the balloon catheter device 310 and is connected with the connecting lead wire M. Using a similar connection method, multiple shock wave electrode assemblies can be arranged inside the balloon to generate shock waves in the axial direction of the first hole of the shock wave electrode assembly 200. Compared with the case of adopting common shock wave electrode assemblies, the threshold voltage, for generating the shock waves, of the shock wave electrode assembly of the balloon catheter device according to the embodiment of the present disclosure is lower than that of a common shock wave electrode assembly, which lowers requirements of the shock wave electrode assembly on pulse voltage and improves the safety of the use of the balloon catheter device; and under the same pulse voltage, the shock wave electrode assembly with the protruding pin structure can generate higher intensity shock wave.

In one embodiment, a plurality of shock wave electrode assemblies 200 are arranged in the same circumferential direction of the inner tube 320, or are arranged at an angle in the circumferential direction. The shock wave is generated at the gap between the outer electrode 230 and the protruding pin 212. For a balloon catheter device with a plurality of shock wave electrode assemblies 200, the relative arrangement direction of the shock wave electrode assemblies 200 will affect the intensity and distribution of shock waves generated by the entire balloon. A plurality of shock wave electrode assemblies 200 may be arranged in the same circumferential direction of the inner tube 320 or at an angle in the circumferential direction according to the position of calcified lesions to be treated in the operation, which is not limited by the present disclosure.

Fig. 9B is a schematic diagram of a balloon catheter device with three shock wave electrode assemblies 200 arranged at 90 degrees and at intervals along the circumferential direction of the inner tube 320, that is, the projections of the three shock wave electrode assemblies on the projection plane perpendicular to the central axis of the inner tube are not coincident. Fig. 9C is a schematic diagram of the balloon catheter device with three shock wave electrode assemblies 200 facing the same direction in the circumferential direction and arranged at a certain distance in the axial direction, that is, the projections of the three shock wave electrode assemblies on the projection plane perpendicular to the central axis of the inner tube coincide. In the embodiment of the present disclosure, a plurality of shock wave electrode assemblies are adopted to enable the balloon catheter device to treat a plurality of calcified lesions at the same time. In addition, the positions of a plurality of shock wave electrode assemblies 200 can be arranged correspondingly according to the positions of the calcified lesions to be treated, such as in the same circumferential direction or at intervals of a certain angle, so as to improve the treatment effect on the blocked lesions, save the operation time, and improve the efficiency.

As mentioned above, the inventor also proposed a medical apparatus, which includes a high-voltage generator and a balloon catheter device 310, a proximal end of a lead wire 340 of the balloon catheter device 310 is connected with the high-voltage generator. In order to ensure the normal operation of the shock wave electrode assembly 200 in the balloon catheter device 310, the high-voltage generator needs to meet the following parameters: the pulse voltage of the high-voltage generator is in a range from 500 V to10 kV, and the pulse voltage width is in a range from 200 ns to 20 µs, the pulse current of the high-voltage generator is in a range from 50 A to 400 A, and the pulse current width is in a range from 10 ns to 2 µs. In one embodiment, the sound pressure intensity of the shock wave generated by each shock wave electrode assembly is in a range from 2 MPa to 20 MPa, and the discharge frequency is in a range from 0.1 Hz to 10 Hz. The shock wave generated by the shock wave electrode assembly propagates through the liquid inside the balloon and impacts the balloon wall and calcified area. Repeated pulses can destroy the structure of calcified lesions and expand narrowed blood vessels without damaging the surrounding soft tissues, which can avoid the problem of blood vessel wall damage caused by balloon expansion in traditional angioplasty. Therefore, the medical apparatus of the embodiment of the present disclosure can more effectively destroy the structure of the calcified lesion attached to the diseased blood vessel wall, and improve the therapeutic effect on the blocked lesion.

The shock wave electrode assembly of the present disclosure has a lower threshold pulse voltage and can complete discharge at a lower pulse voltage, thereby reducing the requirements of the shock wave electrode assembly on pulse voltage. Compared with the common shock wave electrode assembly, at the same pulse voltage, the shock wave electrode assembly of the present disclosure has higher electrical energy density, thereby being able to generate higher energy shock waves.

The following points need to be explained.
(1) The drawings of the embodiment of present disclosure only relate to the structures involved in the embodiment of present disclosure, and other structures can refer to the general design.
(2) Without conflict, the embodiments of the present disclosure and the features in the embodiments may be combined with each other to obtain new embodiments.

The above is only the specific implementation of present disclosure, but the protection scope of present disclosure is not limited to this, and the protection scope of present disclosure shall be subject to the protection scope of the claims.

## Claims

1. A shock wave electrode assembly, **characterized in that**, comprising:
an inner electrode, an outer electrode, and an insulating layer located between the inner electrode and the outer electrode;
wherein a surface of the inner electrode is provided with a protruding pin,
the insulating layer is provided with a first hole,
the outer electrode is provided with a second hole,
a diameter of the second hole is larger than that of the first hole,
the protruding pin, the first hole, and the second hole are configured such that the protruding pin extends through the first hole and into the second hole.

2. The shock wave electrode assembly according to claim 1, **characterized in that**, the inner electrode is in a sheet configuration, the insulating layer and the outer electrode are in annular configurations, the outer electrode and the insulating layer are coaxially arranged, and the outer electrode is sleeved on the insulating layer, and the protruding pin, the first hole and the second hole are coaxially arranged.

3. The shock wave electrode assembly according to claim 1, **characterized in that**, a radial dimension of the first hole is equal to the maximum radial dimension of the protruding pin, so that the protruding pin is fixed in the first hole.

4. The shock wave electrode assembly according to claim 1, **characterized in that**, a height of the protruding pin extending in the second hole is 1/3 to 2/3 of a thickness of the outer electrode.

5. The shock wave electrode assembly according to claim 1, **characterized in that**, the protruding pin is cylindrical, frustum-shaped or conical.

6. The shock wave electrode assembly according to claim 1, **characterized in that**, a ratio of a thickness of the inner electrode to a thickness of the outer electrode is in a range from 1: 1 to 5: 1.

7. The shock wave electrode assembly according to claim 1, **characterized in that**, a ratio of a thickness of the insulating layer to a thickness of the inner electrode is in a range from 1: 1 to 1: 2.

8. The shock wave electrode assembly according to claim 1, **characterized in that**, a ratio of a diameter of the first hole to a diameter of the second hole is in a range from 1: 2 to 1: 5.

9. A balloon catheter device, **characterized in that**, comprising a balloon, an inner tube, an outer tube, and the shock wave electrode assembly according to any one of claims 1 to 8; a distal end of the inner tube extends through the balloon and is connected with a distal end of the balloon, and the shock wave electrode assembly is arranged on an exterior of the inner tube, the outer tube is sleeved on the inner tube and connected with a proximal end of the balloon.

10. The balloon catheter device according to claim 9, **characterized in that**, further comprising a lead wire extending along an axial direction of the balloon catheter device, the lead wire comprises a first lead wire and a second lead wire, the first lead wire is connected with the inner electrode, and the second lead wire is connected with the outer electrode.

11. The balloon catheter device according to claim 9, **characterized in that**, the balloon catheter device comprises a plurality of the shock wave electrode assemblies arranged at intervals along an axial direction of the inner tube.

12. The balloon catheter device according to claim 11, **characterized in that**, the plurality of the shock wave electrode assemblies are arranged in series.

13. The balloon catheter device according to claim 11, **characterized in that**, the plurality of the shock wave electrode assemblies are arranged in the same circumferential direction of the inner tube, or are arranged at an angle in the circumferential direction.

14. A medical apparatus, **characterized in that**, comprising a high-voltage generator and the balloon catheter device according to any one of claims 9 to 13, a proximal end of the lead wire of the balloon catheter device is connected with the high-voltage generator, a pulse voltage of the high-voltage generator is in a range from 500 V to 10 kV, a pulse voltage width is in a range from 200 ns to 20 µs, a pulse current of the high-voltage generator is in a range from 50 A to 400 A, and a pulse current width is in a range from 10 ns to 2 µs.

15. The medical apparatus according to claim 14, **characterized in that**, an intensity of a sound pressure of a shock wave generated by each shock wave electrode assembly is in a range from 2 MPa to 20 MPa, and a discharge frequency is in a range from 0.1 Hz to 10 Hz.
